# EUROPEAN PATENT APPLICATION

(11) **EP 2 907 457 A1**
(43) Date of publication of application: **19.08.2015**
(21) Application number: 13844883.2
(22) Date of filing: 11.10.2013
(51) Int. Cl.: A61B 17/12, A61F 2/88

(54) **MEMBER FOR IMPLANTING IN LIVING ORGANISM, STENT, EMBOLIZATION MEMBER, BLOOD VESSEL EXPANSION KIT, AND ANEURYSM EMBOLIZATION KIT**

(30) Priority: 12.10.2012 JP 2012227452
(71) Applicant: Nhk Spring Co., Ltd., Yokohama-shi, Kanagawa 236-0004 (JP)
(72) Inventor: IWATA Kazuo, Yokohama-shi Kanagawa 236-0004 (JP)
(74) Representative: Brevalex
(86) International application number: PCT/JP2013/077722
(87) International publication number: WO 2014/058043

(57) **Abstract**

Provided is an indwelling member in a living body, the member being flexible and having sufficient strength. Also provided are a stent which is flexible and has sufficient strength, an embolization member which has a high embolization effect, a vasodilator kit, and an aneurysm embolization kit. An indwelling member in a living body of the invention is a double coil wound in coiling with a primary coil formed by winding a first wire of a metal in close coiling. The metallic wire has a diameter in the range of 14 µm to 200 µm, inclusive, and consists of a material selected from platinum, gold, palladium, tungsten, tantalum, cobalt, rhodium, titanium, alloys thereof, stainless steel, a nickel alloy, or a molybdenum alloy.

## Description

### TECHNICAL FIELD

The present invention is related to an indwelling member in a living body, stent, an embolization member, vasodilator kit and an aneurysm embolization kit. In particular, the present invention is related to a medical indwelling member in a living body, stents, an embolic member, vasodilator kit and an aneurysm embolization kit.

### BACKGROUND ART

In surgical procedure in recent years, because a large burden on the patient due to conventional abdominal operations, endoscopic operation and catheterization have been positively introduced. For example, a stenting to extend the narrow segment of the blood vessels as a treatment for angina pectoris, and a catheter treatment with an embolic coil as a treatment for aneurysm reduce the burden on the body of the patient and are of particular interest as a therapeutic method enabling early rehabilitation.

In the treatment of an aneurysm, in order to form a place in the blood vessel for introducing an embolic coil to the aneurysm, a method of treatment combined with stents is being introduced (Patent Literature 1). After a general stent is introduced via a catheter into a blood vessel in a folded mesh tubular structure, the stent is fixed to the blood vessel by expansion and restored by a balloon. A stent graft covered with artificial blood vessels is used in the stent in this case. In embolization which introduces the embolic coil to the aneurysm, it is necessary to introduce a coil of a necessary and sufficient length according to the size of the aneurysm, and when the length of the coil is insufficient, the aneurysm embolization effect is insufficient. On the other hand, when the length of the coil is too long, there is a risk that the coil may protrude into the vessels forming blood clots.

In addition, although a mesh shaped stent can be applied to vessels of a relatively large diameter, presently it is difficult to apply when placing in relatively thin blood vessels such as a brain aneurysm, and therefore smaller stents are desired. In a mesh shaped stent, it is possible to damage the blood cells when blood impinges on the site of the bent mesh. In addition, this type of risk may also produce expanded stenosis in blood vessels due to stenting. As in arteriosclerosis obliterans of the lower extremities, in stenting for sites associated with motor function, in a conventional mesh shaped stent, there is also a risk of damage during exercise and therefore the development of stents with sufficient flexibility and strength is desired.

Examples of stents used in embolization of an aneurysm using a coil shaped structure are reported in Patent Literature 2. However, even in Patent Literature 2, it is not possible to solve the problems due to the use of embolic coils.

### CITATION LIST

### PATENT LITERATURE

Patent Literature 1: Japanese Laid Open Patent: No 2012-055719
Patent Literature 2: Japanese Laid Open Patent: No H10-71154

### SUMMARY OF INVENTION

### TECHNICAL PROBLEM

The present invention aims to solve the aforementioned problems by providing an indwelling member in a living body having sufficient strength and flexibility. In addition, the present invention provides a stent having sufficient strength and flexibility, an embolus member having high embolization effects, an aneurysm embolization kit and vasodilation kit.

### SOLUTION TO PROBLEM

According to one embodiment of the present invention, an indwelling member in a living body is provided including a double coil wound in close coiling with a primary coil formed by winding a first wire of a metal in close coiling.

In the indwelling member in a living body, the first wire may be selected from a platinum, gold, palladium, tungsten, tantalum, cobalt, rhodium, titanium, or alloy thereof, stainless steel, a molybdenum alloy or nickel alloy the first wire has a wire diameter of 14 µm or more and 200 µm or less.

In the indwelling member in a living body, the double coil may have a shape with a diameter expanded toward both end parts from central part.

In the indwelling member in a living body, the inner space of the primary coil may include a second wire of metal disposed along the primary coil.

According to another embodiment of the present invention, a stent is provided including a primary coil formed by winding a first wire of metal in close coiling, the primary coil inserted into a blood vessel to form a double coil wound in close coiling.

In the stent, the first wire may be selected from a platinum, gold, palladium, tungsten, tantalum, cobalt, rhodium, titanium, or alloy thereof, stainless steel, a molybdenum alloy or nickel alloy, the first wire has a wire diameter of 14 µm or more and 200 µm or less.

In the stent, the double coil may have a shape with a diameter expanded toward both end parts from central part.

In the stent, the inner space of the primary coil may include a second wire of metal disposed along the primary coil.

According to another embodiment of the present invention, an embolus member is provided including a primary coil formed by winding a first wire of metal in close coiling, the primary coil inserted into a blood vessel to form a double coil wound in close coiling.

In the embolic element, wire, the first wire may be selected from a platinum, gold, palladium, tungsten, tantalum, cobalt, rhodium, titanium, or alloy thereof, stainless steel, a molybdenum alloy or nickel alloy, the first wire has a wire diameter of 14 µm or more and 200 µm or less.

In the embolus in the member, the diameter of a double coil may be 3 mm or less.

In the embolic element, the double coil may have a shape with a diameter expanded toward both end parts from central part.

In the embolus member, the inner space of the primary coil may include a second wire of metal disposed along the primary coil.

According to another embodiment of the present invention, a vasodilation kit is provided including a primary coil formed by winding a first wire of metal in close coiling, a storage part storing the primary coil, a release means for releasing the primary coil from the storage part, and an operation part for operating the release means, wherein the primary coil is released from the storage part, a double coil is formed by winding in close coiling with the primary coil and the double coil is placed within a blood vessel.

According to another embodiment of the present invention, an aneurysm embolization kit is provided including a primary coil formed by winding a first wire of metal in close coiling, a storage part storing the primary coil, a release means for releasing the primary coil from the storage part, and an operation part for operating the release means, wherein the primary coil is released from the storage part, a double coil is formed by winding in close coiling with the primary coil and the double coil is placed within a blood vessel.

### ADVANTAGEOUS EFFECTS OF INVENTION

In accordance with the present invention, it is possible to provide an indwelling member in a living body having sufficient strength and flexibility. Furthermore, according to the present invention, it is possible to provide a stent having sufficient strength and flexibility, an embolus member with a high embolic effect, an aneurysm embolization kit and vasodilation kit.

### BRIEF DESCRIPTION OF DRAWINGS

Fig. 1 is a schematic diagram showing an indwelling member in a living body 100 related to one embodiment of the present invention;
Fig. 2 is a cross-sectional view along the center line AA' shown in Fig. 1 of the indwelling member in a living body 100 related to one embodiment of the present invention, (a) is a cross-sectional view of an indwelling member in a living body 100 and (b) shows an enlarged view of the part indicated by B in (a);
Fig. 3 is a schematic diagram of an indwelling member in a living body 100 related to one embodiment of the present invention showing a state where it is placed in a part of an aneurysm 60 in a blood vessel 50;
Fig. 4 is a diagram showing a further expanded view of the part shown by B in Fig. 2 (a) of an indwelling member in a living body 100 related to one embodiment of the present invention, (a) shows a cross section shape of the indwelling member in a living body 100 that is placed in a relatively straight blood vessel and (b) shows a cross section shape of the indwelling member in a living body 100 that is placed in a blood vessel which is bent as shown by the arrow;
Fig. 5 is a schematic diagram showing an indwelling member in a living body 200 related to one embodiment of the present invention;
Fig. 6 is a schematic diagram showing an indwelling member in a living body 300 related to one embodiment of the present invention; and
Fig. 7 is a schematic diagram showing an example of an indwelling member in a living body 100 related to one embodiment of the present invention as an embolic member.

### DESCRIPTION OF EMBODIMENTS

An indwelling member in a living body, a stent, an embolic member, a vasodilation kit and an aneurysm embolization kit related to one embodiment of the present invention are described below while referring to the diagrams. Furthermore, the following embodiments are an example of the indwelling member in the living body, stent, embolization member, vasodilation kit and aneurysm embolization kit of the present invention, and the indwelling member in the living body, stent, embolization member, vasodilatation kit and aneurysm embolization kit of the present invention are not limited to the following embodiments.

As a result of intensive studies to solve the problems described above, the present inventors have found that it is possible to realize a stent having sufficient flexibility and strength and an embolus member having a high embolic effect by using an indwelling member in a living body formed with a double coil. In addition, it was found that when the indwelling member in a living body formed with the double coil is used, it is possible to manufacture a small embolic member and realize an embolic member which is placed in small vessels such as blood vessels in the brain.

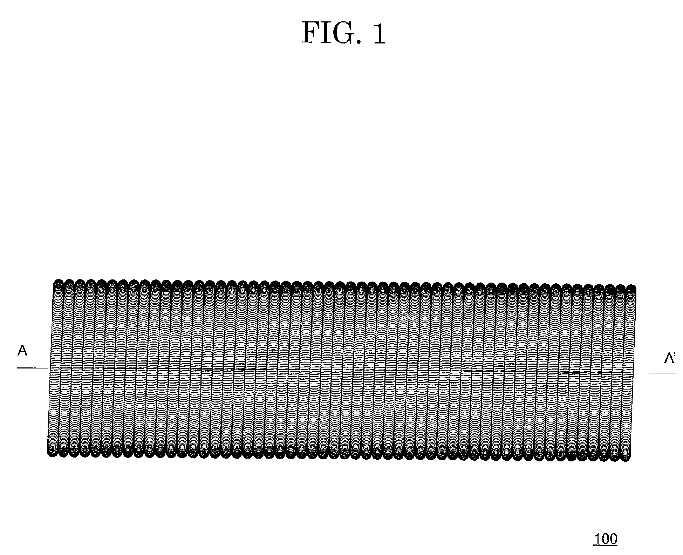

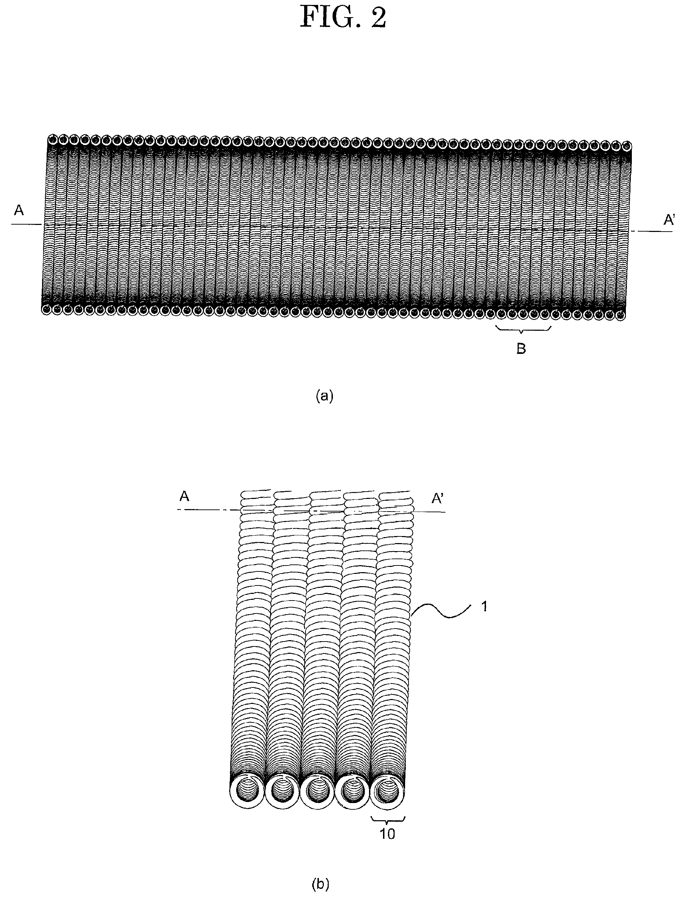

Fig. 1 is a schematic diagram of an indwelling member in a living body 100 related to one embodiment of the present invention. Fig. 2 is a cross-sectional view of the indwelling member in a living body 100 at the center line AA' shown in Fig. 1, Fig. 2 (a) shows a cross-sectional view of the indwelling member in a living body 100 and Fig. 2 (b) is an expanded view of a part indicated by B in Fig. 2 (a). The indwelling member in a living body 100 is a double coil that is wound in close coiling with a primary coil 10 formed by winding a first wire 1 of a metal in close coiling.

In the present embodiment, the metal used for the wire 1 which forms the indwelling member in a living body 100 is not particularly limited as long as it is a metal that has been approved for medical use in stents, and is selected from, for example, platinum, gold, palladium, tungsten, tantalum, cobalt, rhodium, titanium, alloys thereof, stainless steel, nickel alloy or molybdenum alloy. As described above, since the indwelling member in a living body 100 is a double coil that is wound in close coiling with the primary coil 10 formed by winding the metal wire 1 in close coiling, a material which has an elastic modulus and hardness to be wound in this way having corrosion resistance within a living body or organism is used.

Since the indwelling member in a living body 100 is a double coil wound in close coiling with the primary coil 10 formed by the metal wire 1, a diameter of the wire 1 is preferred to be 14 µm or 200 µm or less. When the diameter becomes narrower than 14 µm, it is not possible obtain sufficient strength and the structure described above cannot be formed. In addition, when the diameter exceeds 200 µm, the diameter of the double coil increases and it is difficult to place in a blood vessel.

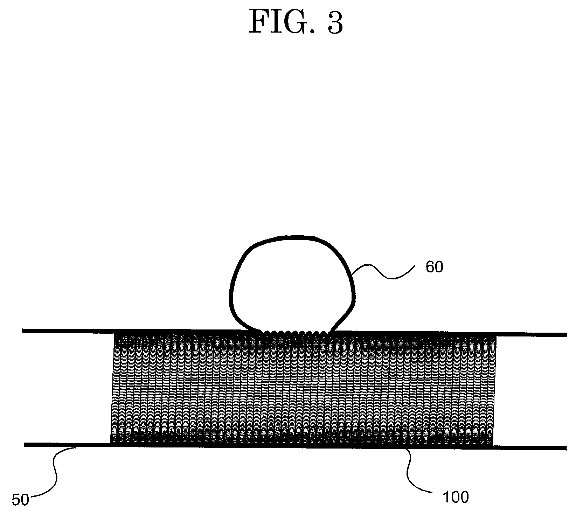

In addition, as described below, in order to be placed in a blood vessel via a catheter, the indwelling member in a living body 100 is stored in the catheter in a state unwound to the primary coil 10, and is inserted through a catheter into position in the blood vessel to take the form of double coil. Fig. 3 is a schematic diagram showing a state in which the indwelling member in a living body 100 is placed in the part of the aneurysm 60 in the vessel 50. Since the indwelling member in a living body 100 is a double coil wound in close coiling with the primary coil 10 formed by winding in close coiling with the metal wire 1, the wire 1 in the primary coil 10 is in close coiling and since pairs of primary coils 10 are also in close coiling, it is possible to prevent the flow of a fluid (blood) to the outside, that is, it is possible to prevent blood flowing through the vessel 50 from entering into the aneurysm 60 when passing through the inside of the indwelling member in a living body 100.

The indwelling member in a living body 100 related to the present invention is different to the embolic method using a conventional embolic coil, and it is possible to prevent the inflow of blood to the aneurysm 60 by being placed in a part of the aneurysm 60 in the vessel 50. Therefore, it can be expected that the indwelling member in a living body 100 related to the present invention does not require rigorous adjustment of the length of the coil such as the embolic method using the conventional embolic coil and more convenient treatment by a doctor is possible.

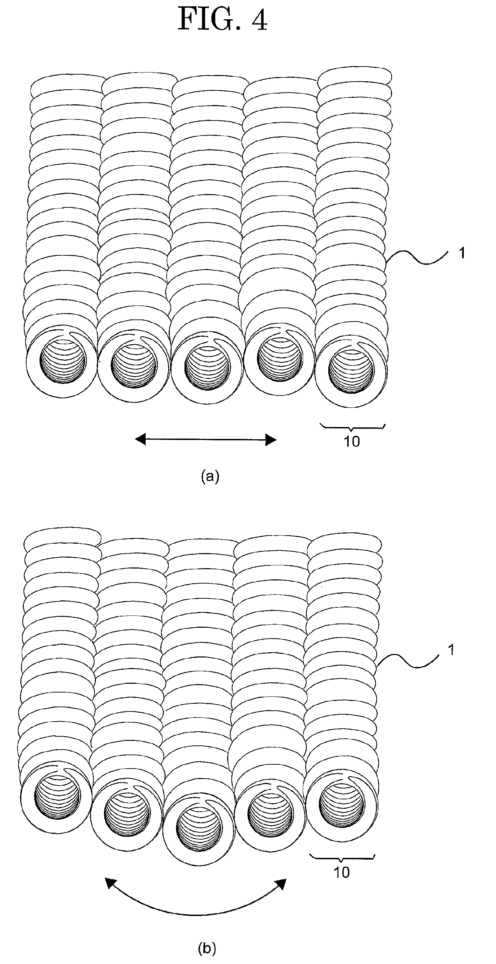

Fig. 4 is a diagram showing a further expanded view of the part shown in Fig. 2 B (a). Fig. 4 (a) shows a cross-section of the indwelling member in a living body 100 which is placed in a relatively straight blood vessel and Fig. 4 (b) shows a cross-section of the indwelling member in a living body 100 which is placed in a blood vessel which is bent as indicated by the arrow. Since the indwelling member in a living body 100 is a double coil wound in close coiling with the primary coil 10 formed by winding in close coiling with the metal wire 1, the corners are not present in the interior of the indwelling member in a living body 100, therefore, it is difficult for blood flowing inside to be damaged. In addition, even when the indwelling member in a living body 100 is placed in a bent blood vessel, as shown in Fig. 4 (b), since pairs of primary coils 10 are bend to be in close coiling within the interior of the indwelling member in a living body 100, it is possible to prevent the inflow of blood into the aneurysm 60.

Since the indwelling member in a living body 100 related to the present invention is a double coil wound in close coiling with the primary coil 10 formed in the metal wire 1, the indwelling member in a living body 100 has flexibility and sufficient strength and can be used as a stent, embolic member, vasodilation kit and aneurysm embolization kit.

### (Modified Example 1)

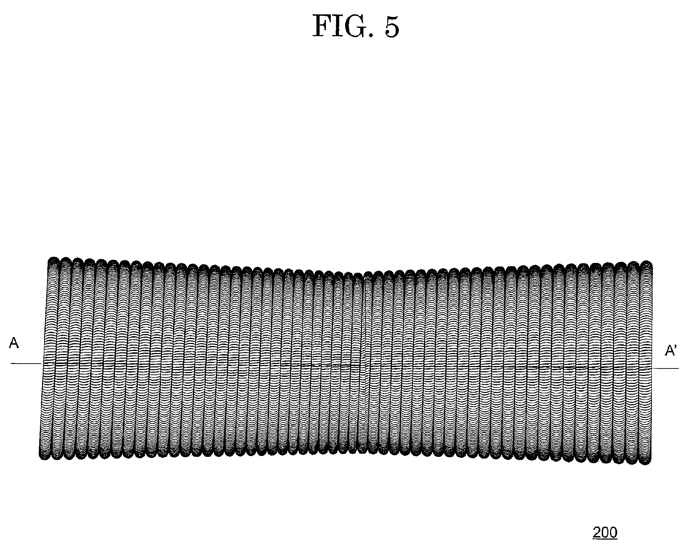

The indwelling member in a living body related to the present invention has a modification which further improves reliability when placed in the vessel 50 in addition to the structure described above. Fig. 5 is a schematic diagram showing a modified example 200 the indwelling member in a living body. The indwelling member in a living body 200 has a double coil with a shape whose diameter expands toward both ends from the central part.

The indwelling member in a living body 200 may be securely fixed by the position of a predetermined blood vessel 50 by providing a shape in which the diameter of both ends is expanded. By having such a structure, as in the treatment of arteriosclerosis obliterans of the lower extremities, even if the indwelling member in a living body 200 is placed at a site associated with motor function, deviation of the indwelling member in a living body due movement can be prevented. In addition, as described above, because the indwelling member in a living body related to the present invention has flexibility and sufficient strength, it is possible to use the indwelling member in a living body suitably as an embolus member and a stent that expands blood vessels of the region associated with motor function.

### (Modified Example 2)

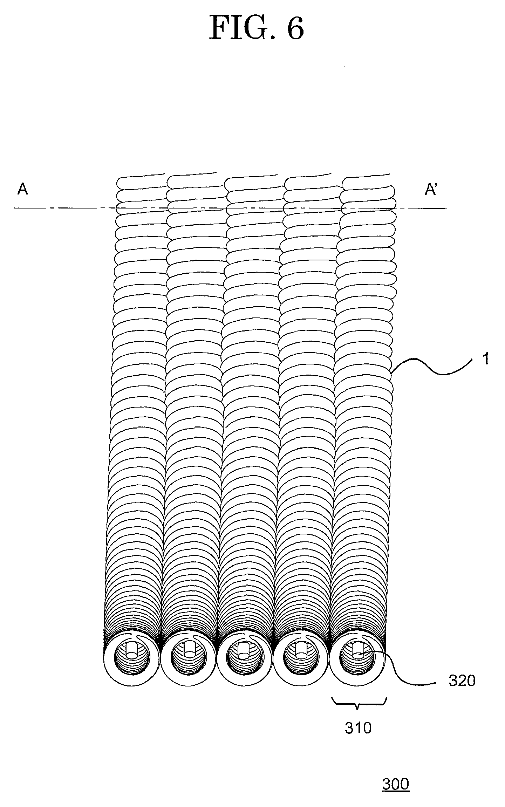

Fig. 6 shows another modified example 300 of the indwelling member in a living body related to the present invention. The indwelling member in a living body 300 has a metal wire (second wire) 320 arranged along the primary coil 310 in an inner space of the primary coil 310 formed by the metal wire 10 (first wire). The first wire 10 and the second wire 320, as described by the indwelling member in a living body 100, is not particularly limited as long as it is a metal that has been approved for medical use in stents, for example, and is selected from platinum, gold, palladium, tungsten, tantalum, cobalt, rhodium, titanium, alloys thereof, stainless steel, nickel alloy or molybdenum alloy. In addition, since the indwelling member in a living body 300 is a double coil wound in close coiling with the primary coil 310 formed by winding in close coiling the metal wire 1, a material having elastic modulus and hardness which can be wound and which has corrosion resistance is used for the first wire 10 and the second wire 320. Furthermore, the first wire 10 and the second wire 320 may also be the same material but may also be different materials.

By providing the indwelling member in a living body 300 with the second wire 320 arranged along the primary coil 310 in an internal space of the primary coil 310 formed by the first wire 10, when the indwelling member in a living body 300 is inserted into the blood vessel 50 from within a catheter, it recovers with high performance in the shape of a double coil which is wound in close coiling with primary coil 310, and it is possible to prevent blood flowing through the blood vessel 50 from entering the aneurysm 60 when passing through the interior of the indwelling member in a living body 300. In addition, the indwelling member in a living body 300 may be a double coil with a shape whose diameter expands toward both ends from the central part the same as the indwelling member in a living body 200.

### (Manufacturing Method)

A method for manufacturing the indwelling member in a living body 100 described above is explained below. The primary coil 10 is molded by pressing a wire to a coiling pin so that the primary coil 10 becomes a predetermined inner diameter. At this time, the coil is tightly wound having an initial tension by forming the coil pitch so that the initial tension is applied to the coil pitch direction. A double coil is similarly molded by pressing the primary coil 10 to the coiling pin so that it has a predetermined inner diameter. At this time, the coil is tightly wound having an initial tension by forming the coil pitch so that the initial tension is applied to the coil pitch direction.

In order to house the indwelling member in a living body 100 in the interior of the catheter, the indwelling member in a living body 100 is housed by unwinding to the primary coil 10. In addition, in the case of manufacturing the indwelling member in a living body 300, after forming a primary coil 310, inserting the second wire 320 into the primary coil 310 and molding a secondary coil, the indwelling member in a living body 300 can be obtained.

### (Aneurysm embolization kit and Vasodilation kit)

Since the indwelling member in a living body related to the present invention described above is a double coil wound in close coiling with the primary coil 10 formed using the metal wire 1, the indwelling member in a living body has flexibility and sufficient strength and can be used as a stent, embolic member, aneurysm embolization kit and vasodilation kit. The aneurysm embolization kit and vasodilation kits related to the present embodiment includes a storage part for storing the primary coil 10 and a release means for releasing the primary coil 10 from the storage part, and an operation part for operating the discharge means, and the primary coil is released from the storage part to form the double coil 100 which is wound in close coiling with the primary coil 10 and the double coil 100 is placed within blood vessel.

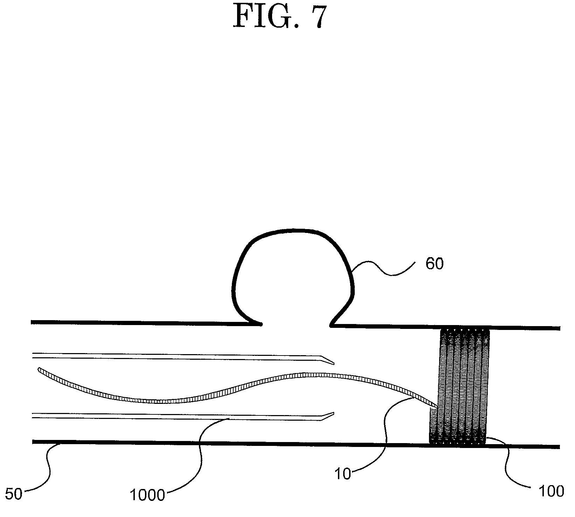

As an example of the vasodilation kit and aneurysm embolization kit, an example of using the indwelling member in a living body 100 as an embolic member is shown in Fig. 7. As described above, the double coil once formed is housed in a catheter 1000 by being unwound to the primary coil 10. The catheter 1000 is inserted within a blood vessel 50 at a certain position away from the position where the aneurysm 60 occurred. An X-ray device or end tip part of the catheter 1000 is introduced to a position up to the position where the aneurysm 60 occurred and the indwelling member in a living body 100 of the primary coil 10 shape that is housed in the catheter 1000 is inserted into the blood vessel 50. The primary coil 10 which is inserted into the vessel 50 is restored to the double coil and is placed in the part of the aneurysm 60 in the blood vessel 50 as an embolic member.

The indwelling member in a living body 100 may be placed in the blood vessel 50 as a stent for expanding the blood vessels using a similar treatment. In the case where the indwelling member in a living body 100 is used as an embolic member for cerebral aneurysm embolization or a stent to be placed in a peripheral vascular, the diameter of the double coil is preferred to be 3 mm or less. The indwelling member in a living body 100 having such a small diameter is suitable for these treatments. In addition, since the indwelling member in a living body 100 is a double coil wound in close coiling with the primary coil 10 formed by winding in close coiling with the metal wire 1, although the indwelling member in a living body 100 related to the present invention can prevent the flow of blood into the aneurysm 60 by itself, this does not exclude it from being used in combination with a conventional artificial blood vessel. For example, in the case where the blood vessels at a stored location become hard or easily torn due to vulnerability, the combined use of artificial blood vessels is preferred.

In the case where the indwelling member in a living body 100 is used as a stent or an embolic member, the indwelling member in a living body 100 is manufactured and provided as an aneurysm embolization kit or vasodilation kit in which the double coil 100 formed once as described above is unwound to the primary coil 10, housed in the catheter 1000 and sterilized by gamma rays, etc. In addition, although the indwelling member in a living body 100 was used as an example, the indwelling member in a living body 200 and 300 described above can also be applied as a stent, a vasodilation kit and an embolic element aneurysm embolization kit related to the present invention. As described above, according to the present invention, it is possible to provide an indwelling member in a living body having flexibility and sufficient strength. In addition, according to the present invention, it is possible to provide a stent having flexibility and sufficient strength, embolus member with high embolic effects, a vasodilation kit and an aneurysm embolization kit.

### REFERENCE SIGNS LIST

- 1:: wire
- 10:: primary coil
- 50:: blood vessel
- 60:: aneurysm
- 100:: indwelling member in a living body
- 200:: indwelling member in a living body
- 300:: indwelling member in a living body
- 310:: primary coil
- 320:: second wire
- 1000:: catheter

## Claims

1. An indwelling member in a living body comprising:
a double coil wound in close coiling with a primary coil formed by winding a first wire of a metal in close coiling.

2. The indwelling member in a living body according to claim 1, wherein the first wire is selected from a platinum , gold, palladium, tungsten, tantalum, cobalt, rhodium, titanium, or alloy thereof, stainless steel, a nickel alloy or a molybdenum alloy , the first wire having a wire diameter of 14 µm or more and 200 µm or less.

3. The indwelling member in a living body according to claim 1 or 2, wherein the double coil has a shape with a diameter expanded toward both end parts from central part.

4. The indwelling member in a living body according to any one of claims 1 to 3, wherein the inner space of the primary coil includes a second wire of metal disposed along the primary coil.

5. A stent comprising:
a primary coil formed by winding a first wire of metal in close coiling, the primary coil inserted into a blood vessel to form a double coil wound in close coiling.

6. The stent according to claim 5, wherein the first wire is selected from a platinum, gold, palladium, tungsten, tantalum, cobalt, rhodium, titanium, or alloy thereof, stainless steel, a molybdenum alloy or nickel alloy, the first wire having a wire diameter of 14 µm or more and 200 µm or less.

7. The stent according to claim 5 or 6, wherein the double coil has a shape with a diameter expanded toward both end parts from central part.

8. The stent according to any one of claims 5 to 7, wherein the inner space of the primary coil includes a second wire of metal disposed along the primary coil.

9. An embolus member comprising:
a primary coil formed by winding a first wire of metal in close coiling, the primary coil inserted into a blood vessel to form a double coil wound in close coiling.

10. The embolus member according to claim 9, wherein the first wire is selected from a platinum, gold, palladium, tungsten, tantalum, cobalt, rhodium, titanium, or alloy thereof, stainless steel, a molybdenum alloy or nickel alloy, the first wire having a wire diameter of 14 µm or more and 200 µm or less.

11. The embolus member according to claim 9, wherein a diameter of the double coil is 3 mm or less.

12. The embolus member according to any one of claims 9 to 11, wherein the double coil has a shape with a diameter expanded toward both end parts from central part.

13. The embolus member according to any one of claims 9 to 12, wherein the inner space of the primary coil includes a second wire of metal disposed along the primary coil.

14. A vasodilation kit comprising:
a primary coil formed by winding a first wire of metal in close coiling;
a storage part storing the primary coil,
a release means for releasing the primary coil from the storage part; and
an operation part for operating the release means;
wherein
the primary coil is released from the storage part, a double coil is formed by winding in close coiling with the primary coil and the double coil is placed within a blood vessel.

15. An aneurysm embolization kit comprising:
a primary coil formed by winding a first wire of metal in close coiling;
a storage part storing the primary coil,
a release means for releasing the primary coil from the storage part; and
an operation part for operating the release means;
wherein
the primary coil is released from the storage part, a double coil is formed by winding in close coiling with the primary coil and the double coil is placed within a blood vessel.
